# EUROPEAN PATENT SPECIFICATION

(11) **EP 2 348 049 B1**
(45) Date of publication and mention of the grant of the patent: **02.10.2013**
(21) Application number: 09819399.8
(22) Date of filing: 09.10.2009
(51) Int. Cl.: G01N 33/564

(54) **APPLICATION OF AIMP1 POLYPEPTIDE**
ANWENDUNG DES AIMP1-POLYPEPTIDS
UTILISATION DU POLYPEPTIDE AIMP1

(30) Priority: 10.10.2008 KR 20080099782
(43) Date of publication of application: 27.07.2011
(73) Proprietor: SNU R&DB Foundation, Seoul 151-742 (KR)
(72) Inventor: KIM, Sunghoon, Seoul 135-536 (KR); HAN, Jung Min, Seoul 151-774 (KR)
(74) Representative: Gulde Hengelhaupt Ziebig & Schneider
(86) International application number: PCT/KR2009/005778
(87) International publication number: WO 2010/041892

(56) References cited:
- EP-A1- 1 384 486
- WO-A1-01/95927
- WO-A1-2007/100058
- WO-A1-2008/007818
- WO-A1-2008/133359
- US-B1- 6 228 837
- GU J ET AL: "CLUES TO PATHOGENESIS OF SPONDYLOARTHROPATHY DERIVED FROM SYNOVIAL FLUID MONONUCLEAR CELL GENE EXPRESSION PROFILES", JOURNAL OF RHEUMATOLOGY, JOURNAL OF RHEUMATOLOGY PUBLISHING COMPANY, CA, vol. 29, no. 10, 1 October 2002 (2002-10-01), pages 2159-2164, XP008029647, ISSN: 0315-162X
- ARIANNA BOTTONI ET AL: "Proteasomes and RARS modulate AIMP1/EMAP II secretion in human cancer cell lines", JOURNAL OF CELLULAR PHYSIOLOGY, vol. 212, no. 2, 1 August 2007 (2007-08-01), pages 293-297, XP55019487, ISSN: 0021-9541, DOI: 10.1002/jcp.21083 -& DATABASE UniProt [Online] 24 January 2006 (2006-01-24), "AIMP1_HUMAN", XP002669672, Database accession no. Q12904
- KO, Y. G. ET AL.: 'A cofactor oftRNA synthetase, p43, is secreted to up-regulated proinflammatory genes.' JOURNAL OF BIOLOGICAL CHEMISTRY vol. 276, no. 25, 05 April 2001, pages 23028 - 23033, XP008120067
- HAN, J. M. ET AL.: 'Aminoacyl-tRNA synthetase-interacting multifunctional protein 1/p43 controls endoplasmic reticulum retention of heat shock protein gp96:its pathological implications in lupus-like autoimmune disease.' AMERICAN JOURNAL OF PATHOLOGY. vol. 170, no. 6, June 2007, pages 2042 - 2054, XP008136864
- KIM, E. ET AL.: 'AIMP1/p43 protein induces the maturation of bone marrow-derived dendritic cells with T-helper type 1-polarizing ability.' JOURNAL OF IMMUNOLOGY vol. 180, no. 5, 01 March 2008, pages 2894 - 2902, XP008136870
- KIM. T. S. ET AL.: 'Gene transfer of AIMP1 and B7. 1 into epitope-loaded, fibroblasts induces tumor-specific CTL immunity, and prolongs the survival period of tumor-bearing mice.' VACCINE. vol. 26, no. 47, 13 September 2008, pages 5928 - 5934, XP025613276

<110> SNU R&DB FOUNDATION
<120> Composition for diagnosing rheumatoid arthritis comprising an antibody against AIMP1 polypeptide
<130> LOP09-0038
<160> 1
<170> KopatentIn 1.71
<210> 1
   <211> 312
   <212> PRT
   <213> Homo sapiens
<400> 1
   Met Ala Asn Asn Asp Ala Val Leu Lys Arg Leu Glu Gln Lys Gly Ala
   1               5                   10                  15
   Glu Ala Asp Gln Ile Ile Glu Tyr Leu Lys Gln Gln Val Ser Leu Leu
               20                  25                  30
   Lys Glu Lys Ala Ile Leu Gln Ala Thr Leu Arg Glu Glu Lys Lys Leu
           35                  40                  45
   Arg Val Glu Asn Ala Lys Leu Lys Lys Glu Ile Glu Glu Leu Lys Gln
       50                  55                  60
   Glu Leu Ile Gln Ala Glu Ile Gln Asn Gly Val Lys Gln Ile Pro Phe
   65                  70                  75                  80
   Pro Ser Gly Thr Pro Leu His Ala Asn Ser Met Val Ser Glu Asn Val
                   85                  90                  95
   Ile Gln Ser Thr Ala Val Thr Thr Val Ser Ser Gly Thr Lys Glu Gln
               100                 105                 110
   Ile Lys Gly Gly Thr Gly Asp Glu Lys Lys Ala Lys Glu Lys Ile Glu
           115                 120                 125
   Lys Lys Gly Glu Lys Lys Glu Lys Lys Gln Gln Ser Ile Ala Gly Ser
       130                 135                 140
   Ala Asp Ser Lys Pro Ile Asp Val Ser Arg Leu Asp Leu Arg Ile Gly
   145                 150                 155                 160
   Cys Ile Ile Thr Ala Arg Lys His Pro Asp Ala Asp Ser Leu Tyr Val
                   165                 170                 175
   Glu Glu Val Asp Val Gly Glu Ile Ala Pro Arg Thr Val Val Ser Gly
               180                 185                 190
   Leu Val Asn His Val Pro Leu Glu Gln Met Gln Asn Arg Met Val Ile
           195                 200                 205
   Leu Leu Cys Asn Leu Lys Pro Ala Lys Met Arg Gly Val Leu Ser Gln
       210                 215                 220
   Ala Met Val Met Cys Ala Ser Ser Pro Glu Lys Ile Glu Ile Leu Ala
   225                 230                 235                 240
   Pro Pro Asn Gly Ser Val Pro Gly Asp Arg Ile Thr Phe Asp Ala Phe
                   245                 250                 255
   Pro Gly Glu Pro Asp Lys Glu Leu Asn Pro Lys Lys Lys Ile Trp Glu
               260                 265                 270
   Gln Ile Gln Pro Asp Leu His Thr Asn Asp Glu Cys Val Ala Thr Tyr
           275                 280                 285
   Lys Gly Val Pro Phe Glu Val Lys Gly Lys Gly Val Cys Arg Ala Gln
       290                 295                 300
   Thr Met Ser Asn Ser Gly Ile Lys
   305                 310

## Description

### [Technical Field]

The present invention relates to a use of AIMP1 polypeptide, more particularly to a composition for diagnosis of arthritis comprising an antibody against AIMP1, a kit for diagnosis comprising the same, a method for diagnosis of arthritis comprising: acquiring a specimen from a subject; and detecting AIMP1 polypeptide included in the specimen, and an antibody against the AIMP1 polypeptide for diagnosis of arthritis.

### [Background Art]

Arthritis collectively refers to the diseases associated with inflammatory changes to the joint for any reason. It involves loss of cartilage which connects bones so that they can move smoothly. Arthritis is classified into various kinds, including degenerative arthritis or osteoarthritis, rheumatoid arthritis, avascular necrosis of the femur head, traumatic arthritis, tuberculosis arthritis, pyrogenic arthritis, and the like.

Among them, rheumatoid arthritis is a chronic disease primarily featured by inflammations in the joint lining or the synovium. Prolonged rheumatoid arthritis may result in damage to the joint, causing chronic pain, loss of functioning and disability. Rheumatoid arthritis proceeds in three stages. The first stage is characterized by pain, fever, stiffness, redness, and swelling of the synovial lining resulting in swelling around the joint. The second stage is characterized by fast division and growth of cells, i.e. pannus, leading to thickening of the synovium. In the third stage, the inflammatory cells release enzymes that degrade the bone and cartilage, resulting in loss of the form and alignment of the related joint, stronger pain, and loss of motility. The diagnosis of rheumatoid arthritis is process-based and there is no sure method of diagnosing rheumatoid arthritis. Accordingly, a more convenient technique for diagnosis of rheumatoid arthritis is needed.

Also, osteoarthritis (degenerative arthritis) is an arthritis resulting from degenerative changes in cartilage and nearby bones among the components of the joint. It causes acute pain in weight bearing joints, such as hips and knees, difficulty in moving, and deformation of the joints if left untreated for a long time. There are two major causes of osteoarthritis. One is damage to the joint due to excessive load although the cartilage or bone of the joint is normal. The other is the case where the cartilage or bone of the joint is weaker than normal although the load is normal.

Meanwhile, AIMP1 (ARS-interacting multi-functional protein 1), formerly known as the p43 protein, was renamed so by the inventors of the present invention (Sang Gyu Park, et al., Trends in Biochemical Sciences, 30:569-574, 2005). The AIMP1 is a protein consisting of 312 amino acids. It binds to the multi-tRNA synthetase complex (Deutscher, M. P., Method Enzymol, 29, 577-583, 1974; Dang C. V. et al., Int. J. Biochem. 14, 539-543, 1982; Mirande, M. et al., EMBO J. 1, 733-736, 1982; Yang D. C. et al., Curr. Top. Cell. Regul. 26, 325-335, 1985) and thus enhances catalytic activity of the multi-tRNA synthetase (Park S. G. et al., J. Biol. Chem. 274, 16673-16676, 1999). AIMP1 is secreted from various types of cells, including prostate cancer cells, immune cells and transgenic cells, and the secretion is induced by various stimulations such as TNFα and heat shock (Park S. G. et al., Am. J. Pathol., 166, 387-398, 2005; Barnett G. et al., Cancer Res. 60, 2850-2857, 2000). The secreted AIMP1 is known to work on diverse target cells such as monocytes/macrophages, endothelial cells and fibroblast cells. Gu et al. (Gu et al., J. Rheumatology, vol. 29, No. 10, 2159-2164, 2002) compared gene expression profiles between peripheral blood mononuclear cells (PBMC) of normal individuals and synovial fluid mononuclear cells (SFMC) of patients suffering from arthritis. This comparison was done in order to elucidate involvement of certain signaling pathways in the pathogenesis of spondyloarthropathy. In WO 2007/100058 A1 a method of diagnosis of arthritis is disclosed wherein expression level of HSC71 is measured in synovial fluid.

### [disclosure]

### [Technical Problem]

While the inventors of the present invention have studied about the physiological functions of AIMP1 protein, they found out that the level of the AIMP1 protein in synovial fluid is increased in arthritic patients. Based on the finding, they have developed a composition for diagnosis of arthritis comprising AIMP1 polypeptide as an effective ingredient.

Accordingly, the object of the present invention is to provide a use of AIMP1 polypeptide.

### [Technical Solution]

To achieve the above object, the present invention provides an antibody against AIMP1 polypeptide and a composition comprising said antibody both for use in a method for diagnosis of arthritis as defined in the claims.

Hereafter, the present invention will be described in more detail.

A composition of the present invention comprises an antibody against the AIMP1 polypeptide according to SEQ ID NO:1 and is used for diagnosis of arthritis.

AIMP1 polypeptide is known as p43 and consist of 312 of amino acid and binds to multi-tRNA synthetase complex and enhances its catalytic activity. The AIMP1 polypeptide is a polypeptide having an amino acid sequence represented by SEQ ID NO: 1. According to some aspects of the disclosure, the AIMP1 polypeptide may be one disclosed in Genbank Accession No. NM_004755, BC014051, CR542281. According to some aspects of the disclosure, the AIMP1 polypeptide may be functional equivalents thereof.

The term "functional equivalents" refer to polypeptides which have at least 70%, preferably at least 80%, more preferably at least 90% amino acid sequence homology (i.e., identity) with the amino acid sequence represented by SEQ ID NO: 1. For example, it comprises polypeptide having 70%, 71%, 72%, 73%, 74%, 75%, 76%, 77%, 78%, 79%, 80%, 81%, 82%, 83%, 84%, 85%, 86%, 87 %, 88 %, 89%, 90%, 91%, 92%, 93%, 94%, 95%, 96%, 97%, 98%, 99% and 100% of amino acid sequence homology and exhibit substantially identical physiological activity to AIMP1 polypeptide. As used herein, "substantially identical physiological activity" refers showing original activity of AIMP1 polypeptide as well as enhancing catalytic activity by binding to multi-tRNA synthetase complex. The functional equivalents may include, for example peptides produced by as a result of addition, substitution or deletion of some amino acid of SEQ ID NO:1. Substitutions of the amino acids are preferably conservative substitutions. Examples of conservative substitutions of naturally occurring amino acids are as follows: aliphatic amino acids (Gly, Ala, Pro), hydrophobic amino acids (Ile, Leu, Val), aromatic amino acids (Phe, Tyr, Trp), acidic amino acids (Asp, Glu), basic amino acids (His, Lys, Arg, Gln, Asn) and sulfur-containing amino acids (Cys, Met). In addition, the functional equivalents also include variants with deletion of some of the amino acid sequence of the AIMP1 polypeptide. Deletion or substitutions of the amino acids are preferably located at regions that are not directly involved in the physiological activity of the AIMP1 polypeptide. And deletion of the amino acids is preferably located at regions that are not directly involved in the physiological activity of the AIMP1 polypeptide. In addition, the functional equivalents also include variants with addition of several amino acids in both terminal ends of the amino acid sequence of the AIMP1 or in the sequence. Moreover, the functional equivalents also include polypeptide derivatives which have modification of some of the chemical structure of the polypeptide while maintaining the fundamental backbone and physiological activity of the polypeptide. Examples of this modification include structural modifications for changing the stability, storage, volatility or solubility of the AIMP1 polypeptide.

Sequence identity and homology is defined herein as the percentage of amino acid residues in the candidate sequence that are identical with amino acid sequence of AIMP1 polypeptide, after aligning the sequences and introducing gaps, if necessary, to achieve the maximum percent sequence identity, and not considering any conservative substitutions as part of the sequence identity. In addition, none of N-terminal, C-terminal, or internal extensions, deletions, or insertions into the amino acid sequence of AIMP1 polypeptide shall be construed as affecting sequence identity or homology. Thus, sequence identity can be determined by standard methods that are commonly used to compare the similarity in position of the amino acids of two polypeptides. Using a computer program such as BLAST or FASTA, two polypeptides are aligned for optimal matching of their respective amino acids (either along the full length of one or both sequences or along a predetermined portion of one or both sequences). The programs provide a default opening penalty and a default gap penalty, and a scoring matrix such as PAM 250 (a standard scoring matrix; see Dayhoff et al., in Atlas of Protein Sequence and Structure, vol. 5, supp. 3, 1978) can be used in conjunction with the computer program. For example, the percent identity can be calculated as the follow. The total number of identical matches multiplied by 100 and then divided by the sum of the length of the longer sequence within the matched span and the number of gaps introduced into the longer sequences in order to align the two sequences.

An antibody against AIMP1 polypeptide refers a specific protein molecule directing to an antigenic region of AIMP1 polypeptide. For the purpose of the present invention, the antibody refers an antibody specifically binding to AIMP1 polypeptide having an amino acid sequence according to SEQ ID NO:1 and it comprises polyclonal antibody, monoclonal antibody and recombinant antibody.

The antibodies against the AIM1 polypeptide may be easily prepared in accordance with conventional technologies known to one skilled in the art. Polyclonal antibodies may be prepared by a method widely known in the art, which includes injecting the AIM1 polypeptide antigen into an animal and collecting blood samples from the animal to obtain serum containing antibodies. Such polyclonal antibodies may be prepared from a certain animal host, such as goats, rabbits, sheep, monkeys, horses, pigs, cows and dogs.

Monoclonal antibodies may be prepared by a method widely known in the art, such as a hybridoma method (Kohler and Milstein, European Journal of Immunology, 6:511-519(1976)) or a phage antibody library technique (Clackson et al, Nature, 352:624-628(1991); and Marks et al, J. Mol. Biol., 222:58, 1-597(1991)).

The hybridoma method employs cells from an immunologically suitable host animal injected with a diagnostic marker protein of lung cancer as an antigen, such as mice, and a cancer or myeloma cell line as another group. Cells of the two groups are fused with each other by a method widely known in the art, for example, using polyethylene glycol , and antibody-producing cells are proliferated by a standard tissue culture method. After uniform cell colonies are obtained by subcloning using a limited dilution technique, hybridomas capable of producing an antibody specific for the diagnostic marker protein of AIM1 are cultivated in large scale in vitro or in vivo according to a standard technique. Monoclonal antibodies produced by the hybridomas may be used in an unpurified form, but are preferably used after being highly purified by a method widely known in the art so as to obtain best results. The phage antibody library method includes constructing a phage antibody library in vitro by obtaining genes for antibodies ( single-chain fragment variable ( scFv) ) to a variety of intracellular lung cancer markers and expressing them in a fusion protein form on the surface of phages, and isolating monoclonal antibodies binding to lung cancer-specific proteins from the library. Antibodies prepared by the above methods are isolated using gel electrophoresis, dialysis, salting out, ion exchange chromatography, affinity chromatography, and the like.

In addition, the antibodies of the present invention include complete forms having two full-length light chains and two full-length heavy chains, as well as functional fragments of antibody molecules. The functional fragments of antibody molecules refer to fragments retaining at least an antigenbinding function, and include Fab, F(ab'), F(ab')2 and Fv.

An AIM1 polypeptide can be prepared by separating from nature materials or genetic engineering methods. For example, a DNA molecule encoding an AIM1 polypeptide or its functional equivalents is constructed according to any conventional method. The DNA molecule may synthesize by performing PCR using suitable primers. Alternatively, the DNA molecule may also be synthesized by a standard method known in the art, for example using an automatic DNA synthesizer (commercially available from Biosearch or Applied Biosystems). The constructed DNA molecule is inserted into a vector comprising at least one expression control sequence(ex: promoter, enhancer) that is operatively linked to the DNA sequence so as to control the expression of the DNA molecule, and host cells are transformed with the resulting recombinant expression vector. The transformed cells are cultured in a medium and condition suitable to express the DNA sequence, and a substantially pure polypeptide encoded by the DNA sequence is collected from the culture medium. The collection of the pure polypeptide may be performed using a method known in the art, for example, chromatography. In this regard, the term "substantially pure polypeptide" means the inventive polypeptide that does not substantially contain any other proteins derived from host cells. For the genetic engineering method for synthesizing the inventive polypeptide, the reader may refer to the following literatures: Maniatis et al., Molecular Cloning: A Laboratory Manual, Cold Spring Harbor Laboratory 1982; Sambrook et al., Molecular Cloning; A Laboratory Manual, Cold Spring Harbor Press, N.Y., Second(1998) and Third(2000) Editions; Gene Expression Technology, Method in Enzymology, Genetics and Molecular Biology, Method in Enzymology, Guthrie & Fink (eds.), Academic Press, San Diego, Calif. 1991; and Hitzeman et al., J. Biol. Chem., 255, 12073-12080 1990.

Also, the AIM1 polypeptide can be chemically synthesized easily according to any technique known in the art (Creighton, Proteins: Structures and Molecular Principles, W.H. Freeman and Co., NY, 1983). As a typical technique, they are not limited to, but include liquid or solid phase synthesis, fragment condensation, F-MOC or T-BOC chemistry (Chemical Approaches to the Synthesis of Peptides and Proteins, Williams et al., Eds., CRC Press, Boca Raton Florida, 1997; A Practical Approach, Atherton & Sheppard, Eds., IRL Press, Oxford, England, 1989).

Arthritis of the present invention refers to the diseases associated with inflammatory changes to the joint for any reason. For example, it may be degenerative arthritis or osteoarthritis, rheumatoid arthritis, avascular necrosis of the femur head, traumatic arthritis, tuberculosis arthritis and pyrogenic arthritis, and preferably it may be rheumatoid arthritis or degenerative arthritis (osteoarthritis).

Meanwhile, a kit is provided for diagnosis of arthritis comprising an antibody against AIM1 polypeptide.

The kit may further comprise an instrument and/or a reagent for immunological analysis which are well known in the art as well as an antibody against AIM1 polypeptide.

The immunological analysis may comprise a method as long as it can measure binding of antigen-antibody. These methods are well known in the art and for example, there are immunocytochemistry and immunohistochemistry, radioimmunoassays, ELISA (Enzyme Linked Immunoabsorbent assay), immunoblotting, Farr assay, immunoprecipitation, latex cohesion, erythrocyte cohesion, nephelometry, immunodiffusion, count-current electrophoresis, single radical immunodiffusion, protein chip and immunofluorescence.

As an instrument and/or a reagent for immunological analysis, it comprises a suitable carrier or a support, a marker which produces a detectable signal, a diluent and a cleansing agent. In addition, when the marker is an enzyme, it may comprise a substrate which is enable to measure activity of the enzyme and a reaction blocking agent.

An antibody against an AIM1 polypeptide which is included in the kit for diagnosis preferably may be fixed to a suitable carrier or a support as disclosed in the reference (Antibodies: A Laboratory Manual, Harlow & Lane; Cold Spring Harbor, 1988). As examples of a suitable carrier or a support, there are agarose, cellulose, nitrocellulose, dextran, sephadex, sepharose, liposome, carboxymethylcellulose, polyacrylamide, polysterine, gabbro, filter paper, ion exchange resin, plastic film, plastic tube, glass, polyamine-methylvinyl-ether-maleic acid copolymer, amino acid copolymer, ethylene-maleic acid copolymer, nylon, cup, flat packs. As other solid substrates, there are a cell culture plate, a ELISA plate, a tube and a polymeric membrane. The support may have a random form, for example, a form of globular (beads), cylindrical (test tube or inside of well), plane (sheet, test strip).

A marker which produces a detectable signal enables to measure formation of antigen-antibody complex qualitatively and quantitatively and the examples are a enzyme, a fluorescent material, a ligand, a luminous material, microparticle, a redox molecule and a radioactive isotope. As a enzyme, β-glucuronidase, β-D-glucosidase, urase, peroxidase, alkaline phosphatase, acetylcholine esterase, glucose oxidase, hexokinase, malate dehydrogenase, glucose-6-phosphate hydrogenase and invertase may be used. As a fluorescent material, fluorescin, isothiocyanate, rodamin, phycoerythrin, phycocyanin, allophycocyanin, fluorescinisothiocyanate may be used. As a ligand, there are biotin derivatives and as a luminous material, acridium, ester, luciferin and luciferase. As a microparticle, there are colloid gold and colored latex, and as a redox molecule, there are ferrocene, ruthenium complex compound, biologen, quinone, Ti ion, Cs ion, dimide, 1,4-benzoquinone and hydroquinone. As a radioactive isotope, 3H, 14C, 32P, 35S, 36Cl, 51Co, 58Co, 59Fe, 90Y, 125I, 131I and 186Re. However, besides the above mentioned things, anything can be used as long as it can be used in immunological analysis.

Also, the kit for diagnosis of arthritis may further comprise composition for detecting a marker for arthritis which is well known in the art. For example, a marker for arthritis which is well known in the art may be, but not limited thereto, rheumatoid factor, cyclic citrullinated peptide, citrulline antibody, antinuclear antibody and C-reactive protein(Critical reviews in clinical laboratory sciences, vol.44, pp339-363 (2007))

Also, to provide necessary information for diagnosis of arthritis, the present invention provides a method for detecting an AIMP1 polypeptide through antigen-antibody reaction from a sample of a patient. At this time, antigen-antibody reaction is well described above.

Meanwhile, the present disclosure provides a method for diagnosis of arthritis comprising : acquiring a specimen from a subject; and detecting AIMP1 polypeptide included in the specimen.

As used herein, the term "subject" means mammals, particularly animals including human beings. The subject may be patients in need of diagnosis.

As used herein, the term "specimen (or sample)" refers to synovial fluid. The sample is acquired from animals, preferably from mammals and most preferably from human beings. The sample may be pretreated before use. For example, it may comprise filtration, distillation, extraction, concentration, inactivation of inhibitors, adding of a reagent. In addition, the protein may be isolated from the sample and used for detection.

As used herein, the term "detection" means analysis, imaging, identification and deciding of existence or non-existence of a AIMP1 polypeptide and subunits thereof.

Meanwhile, the present invention provides an antibody against a AIMP1 polypeptide for diagnosis of arthritis.

For the genetic engineering method for nucleotides and proteins, it may be referred to the following literatures: Maniatis et al., Molecular Cloning: A Laboratory Manual, Cold Spring Harbor Laboratory 1982; Sambrook et al., Molecular Cloning; A Laboratory Manual, Cold Spring Harbor Press, N.Y., Second(1998) and Third(2000) Editions; Gene Expression Technology, Method in Enzymology, Genetics and Molecular Biology, Method in Enzymology, Guthrie & Fink (eds.), Academic Press, San Diego, Calif. 1991; and Hitzeman et al., J. Biol. Chem., 255, 12073-12080 1990.

### [Advantageous Effects]

Accordingly, the present invention provides a composition for diagnosis of arthritis including an antibody against AIMP1 polypeptide as a novel diagnosis marker of arthritis and an antibody against the AIMP1 polypeptide for diagnosis of arthritis. The composition and antibody against the AIMP1 polypeptide may be used to diagnose arthritis early since they allow easy diagnosis of arthritis using a specimen from the patient.

### [Description of Drawings]

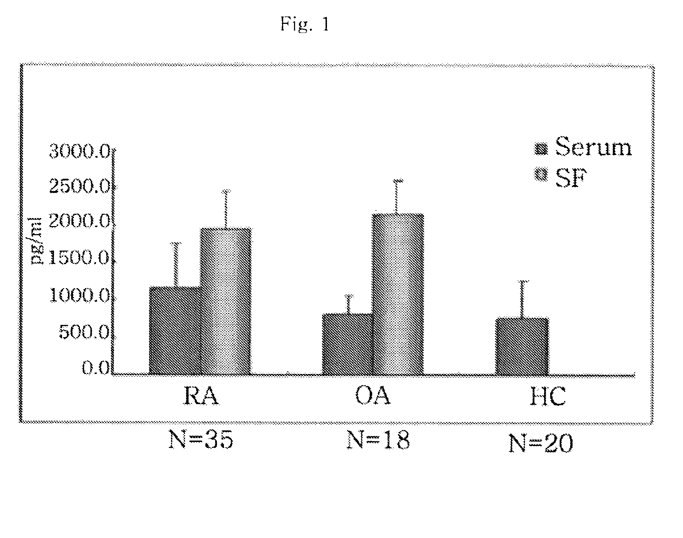

Figure 1 is a graph identifying level of AIMP1 in synovial fluid of the normal group (HC, n=20), rheumatoid arthritis group (RA, n=35) and osteoarthritis patient group (OA, n=18) by ELISA.

### [Mode for Invention]

The examples and experiments will now be described. The following examples and experiments are for illustrative purposes only and not intended to limit the scope of the present invention.

### <Example 1>

### Confirmation of AIMP1 level in specimen from arthritic patient

AIMP1 level in synovial fluid (SF) and serum acquired from 35 patients diagnosed with rheumatoid arthritis (RA), 18 patients diagnosed with osteoarthritis (OA) and 20 healthy people was measured as follows. The synovial fluid was collected from the cartilage tissue of the patients or healthy people by arthrocentesis or joint aspiration using a syringe.

The level of AIMP1 in the collected synovial fluid and serum was measured using a commercially available ELISA kit for human AIMP1 (Cat. No. IMG-100, Imagene, Co. Ltd.) according to the manufacturer's instructions as follows. The specimen (10 µL) was mixed with a diluent (90 µL) and then added to a 96-well ELISA plate. After reaction for 2 hours, the mixture was washed 4 times with washing buffer. Then, peroxidase-conjugated anti-AIMP1 antibody (1 µL) diluted in a diluent (1 mL) was added to a 96-well plate, 100 µL per each well. After reaction for 1 hour and washing 4 times with washing buffer, substrate solution (100 µL) was added to each well. After reaction for 30 minutes, stop solution (100 µL) was added to each well. Then, absorbance was measured at 450 nm to determine the level of AIMP1.

As seen from Fig. 1, AIMP1 was detected in high level in the synovial fluid from the patients with rheumatoid arthritis (RA) or osteoarthritis (OA), whereas it was not detected in the synovial fluid from the healthy people (HC). Accordingly, AIMP1 may be utilized as a marker for rheumatoid arthritis or osteoarthritis. Further, more AIMP1 was detected from the serum of the patients with rheumatoid arthritis than that from the healthy people. There was no significant difference between the osteoarthritic patients and the healthy people.

### [Industrial Applicability]

As can be seen foregoing, the present invention provides a composition for diagnosis of arthritis including an antibody against AIMP1 polypeptide as a novel diagnosis marker of arthritis and an antibody against the AIMP1 polypeptide for diagnosis of arthritis. The composition and antibody against the AIMP1 polypeptide may be used to diagnose arthritis early since they allow easy diagnosis of arthritis using a specimen from the patient.
## Claims

1. An antibody against AIMP1 polypeptide having an amino acid sequence according to SEQ ID NO: 1 for use in a method for diagnosis of arthritis comprising : acquiring a specimen from a subject; and detecting AIMP1 polypeptide in the specimen;
wherein the specimen is synovial fluid and wherein increased level of AIMP1 polypeptide is indicative of arthritis.

2. A composition comprising an antibody of claim 1 for use in a method for diagnosis of arthritis comprising: acquiring a specimen from a subject; and detecting AIMP1 polypeptide in the specimen;
wherein the specimen is synovial fluid and wherein increased level of AIMP1 polypeptide is indicative of arthritis.

3. The composition of claim 2 for use in a method of claim 2, wherein the arthritis is rheumatoid arthritis or osteoarthritis.

4. l An antibody of claim 1 for use in a method of claim 1, wherein the arthritis is rheumatoid arthritis or osteoarthritis.

## Patentansprüche

1. Antikörper gegen das AIMPI-Polypeptid mit einer Aminosäuresequenz gemäß SEQ ID NR: 1 zur Verwerdung in einem Verfahren zur Diagnose von Arthritis, umfassend: Gewinnung einer Probe von einem Lebewesen; und Nachweis des AIMP1-Polypeptids in der Probe;
wobei die Probe Synovialflüssigkeit ist und wobei ein erhöhter Spiegel des AIMP1-Polypeptids Arthritis anzeigt.

2. Zusammensetzung, die einen Antikörper nach Anspruch 1 umfasst, zur Verwerdung in einem Verfahren zur Diagnose von Arthritis, umfassend: Gewinnung einer Probe von einem Lebewesen; und Nachweis des AIMP1 -Polypeptids in der Probe;
wobei die Probe Synovialflüssigkeit ist und wobei ein erhöhter Spiegel des AIMP1-Polypeptids Arthritis anzeigt.

3. Zusammensetzung nach Anspruch 2 zur Verwendung in einem Verfahren nach Anspruch 2, wobei die Arthritis rheumatoide Arthritis oder Osteoarthritis ist.

4. Antikörper nach Anspruch 1 zur Verwendung in einem Verfahren nach Anspruch 1, wobei die Arthritis rheumatoide Arthritis oder Osteoarthritis ist.

## Revendications

1. Anticorps dirigé contre le polypeptide AIMP1 ayant une séquence d'acides aminés selon SEQ ID NO : 1, destiné à être utilisé dans un procédé de diagnostic de l'arthrite comprenant :
l'acquisition d'un spécimen en provenance d'un sujet ; et la détection du polypeptide AIMP1 dans le spécimen ;
le spécimen étant du liquide synovial et l'augmentation du niveau du polypeptide AIMP1 étant symptomatique de l'arthrite.

2. Composition comprenant un anticorps de la revendication 1, destinée à être utilisée dans un procédé de diagnostic de l'arthrite comprenant : l'acquisition d'un spécimen en provenance d'un sujet ; et la détection du polypeptide AIMP1 dans le spécimen ;
le spécimen étant du liquide synovial et l'augmentation du niveau du polypeptide AIMP1 étant symptomatique de l'arthrite.

3. Composition selon la revendication 2, destinée à être utilisée dans un procédé selon la revendication 2, l'arthrite étant la polyarthrite rhumatoïde ou l'arthrose.

4. Anticorps selon la revendication 1, destiné à être utilisé dans un procédé selon la revendication 1, l'arthrite étant la polyarthrite rhumatoïde ou l'arthrose.
